# EUROPÄISCHE PATENTSCHRIFT

(11) **EP 2 825 464 B1**
(45) Veröffentlichungstag und Bekanntmachung des Hinweises auf die Patenterteilung: **12.04.2017**
(21) Anmeldenummer: 13711282.7
(22) Anmeldetag: 19.02.2013
(51) Int. Cl.: B64D 37/32, G01N 27/62, G01N 33/00

(54) **LUFTFAHRZEUG MIT VORRICHTUNG ZUR ÜBERWACHUNG DER SAUERSTOFFKONZENTRATION IN EINEM TREIBSTOFFTANK**
AIRCRAFT WITH DEVICE FOR MONITORING THE CONCENTRATION OF OXYGEN IN A FUEL TANK
AÉRONEF AVEC DISPOSITIF POUR SURVEILLER LA CONCENTRATION EN OXYGÈNE DANS UN RESERVOIR DE CARBURANT

(30) Priorität: 15.03.2012 DE 102012005058
(43) Veröffentlichungstag der Anmeldung: 21.01.2015
(73) Patentinhaber: Airbus Defence and Space GmbH, 85521 Ottobrunn (DE)
(72) Erfinder: MÜLLER, Gerhard, 85567 Grafing (DE); GÖBEL, Johann, 81547 München (DE); HACKNER, Angelika, 81679 München (DE); FRIEDBERGER, Alois, 85667 Oberpframmern (DE)
(74) Vertreter: Isarpatent
(86) Internationale Anmeldenummer: PCT/DE2013/100062
(87) Internationale Veröffentlichungsnummer: WO 2013/135232

(56) Entgegenhaltungen:
- WO-A1-2004/113169
- US-A- 3 154 680
- US-A1- 2007 114 395
- SIMMONDS ET AL: "Electron-capture detector with a photoemissive electron source", JOURNAL OF CHROMATOGRAPHY, ELSEVIER SCIENCE PUBLISHERS B.V, NL, Bd. 399, 1. Januar 1987 (1987-01-01), Seiten 149-164, XP026553815, ISSN: 0021-9673, DOI: 10.1016/S0021-9673(00)96118-1 [gefunden am 1987-01-01]
- TEKI RANGANATH ET AL: "Enhanced photoemission from nanostructured surface topologies", APPLIED PHYSICS LETTERS, AIP, AMERICAN INSTITUTE OF PHYSICS, MELVILLE, NY, US, Bd. 89, Nr. 19, 10. November 2006 (2006-11-10), Seiten 193116-193116, XP012086898, ISSN: 0003-6951, DOI: 10.1063/1.2387970

## Beschreibung

Die Erfindung betrifft ein Luftfahrzeug mit mindestens einem ein Inertgas enthaltenden Treibstofftank und einer Vorrichtung zur Überwachung der Sauerstoffkonzentration.

Es ist aus Flugzeugunglücken bekannt, dass das Vorhandensein von Umgebungsatmosphäre in den Treibstofftanks von Luftfahrzeugen unter extremen Bedingungen zu Explosionen führen kann. Insbesondere bei leeren oder fast leeren Tanks und hohen Umgebungstemperaturen - beispielsweise beim Aufenthalt in den Tropen - bildet das den (fast) leeren Tank ausfüllende verdampfte Kerosin in Verbindung mit dem Luftsauerstoff ein explosionsfähiges Gemisch. Daher sind seit Jahren Bestrebungen im Gange, dieses Risiko zu vermeiden. Eine bevorzugte Lösung besteht darin, den durch die zunehmende Entleerung sich bildenden Raum nicht mit Umgebungsluft sondern mit einem Inertgas zu befüllen. Um nicht durch die Mitführung ausreichender Mengen eines Inertgases das Flugzeuggewicht zu erhöhen, ist es beispielsweise aus der US 2005/0115404 bekannt, flüssigen Stickstoff aus der Umgebungsluft zu gewinnen und dieses inertgas dem sich leerenden Tanks zuzuführen. Um zu gewährleisten, dass die Bedingungen in den Treibstofftanks gesichert sind ist es erforderlich, den Sauerstoffgehalt in den Treibstofftanks zu erfassen, damit gegebenenfalls entsprechende Maßnahmen ergriffen werden können wie vermehrte oder verminderte Stickstofferzeugung oder eine Kühlung des Tankes.

Aus der WO 2004/113169 ist ein gattungsgemäßer Sauerstoffsensor zur Überwachung der Sauerstoffkonzentration in einem ein Inertgas enthaltenden Treibstofftank eines Luftfahrzeuges bekannt, der nach dem Prinzip der Absorptionsspektroskopie arbeitet. Dieses System hat den Nachteil, dass aufgrund der physikalischen Eigenschaften des Messprinzips entweder eine mäßige Messgenauigkeit in Kauf genommen werden muss oder der technische Aufwand im Hinblick auf die Schaltungsgüte sowie Kompensationen von Temperatur- und Druckunterschieden in der Messstrecke sehr hoch ist.

Andere Sensorprinzipien wie Lambda-Sonden erfordern erwärmte Messflächen, was sowohl unter dem Aspekt der Sicherheit als auch der Wirtschaftlichkeit unerwünscht ist.

Die US 2007/0114395 A1 offenbart eine lonen-Mobilitätsspektroskopie (IMS-) Vorrichtung zur Erfassung von Sprengstoffen mit einer Ionisationskammer und einer Lichtimplus-Erzeugungsvorrichtung zur Bestrahlung einer Elektronen-Emitterfläche.

Aufgabe der Erfindung ist es, ein Luftfahrzeug mit mindestens einem ein Inertgas enthaltenden Treibstofftank und einer Vorrichtung zur Überwachung der Sauerstoffkonzentrationbereitzustellen, das baulich einfach eine genaue Messung der Sauerstoffkonzentration ermöglicht. Dabei sollte der Messvorgang möglichst ohne eine Temperaturerhöhung erfolgen.

Die Lösung dieser Aufgabe ergibt sich aus den Merkmalen des Anspruchs 1. Vorteilhafte Weiterbildungen und Ausgestaltungen sind Gegenstand der abhängigen Ansprüche. Weitere Merkmale, Anwendungsmöglichkeiten und Vorteile der Erfindung ergeben sich aus der nachfolgenden Beschreibung, sowie der Erläuterung von Ausführungsbeispielen der Erfindung, die in den Figuren dargestellt sind.

Insbesondere wird die Aufgabe durch folgende Merkmale gelöst:
- eine Ionisationskammer, die mit dem Treibstofftank kommuniziert und von einem mit Inertgasstrom aus dem Treibstofftank durchströmbar ist;
- wobei ein Ende der Ionisationskammer eine Elektronen-Emitterfläche aufweist;
- wobei die Ionisationskammer eine der Elektronen-Emitterfläche gegenüberliegende Kollektorfläche aufweist, die mit einer Ladungsträger-Detektionsvorrichtung gekoppelt ist;
- eine Lichtimpuls-Erzeugungsvorrichtung zur Bestrahlung der Elektronen-Emitterfläche zur Erzeugung von Elektronen in der Ionisationskammer;
- wobei die Ionisationskammer von einer Felderzeugungseinrichtung umgeben ist zur Erzeugung eines elektrischen Feldes, um negative Ladungsträger in Richtung zur Kollektorfläche zu bewegen;
- eine Steuereinheit, welche die Lichtimpuls-Erzeugungsvorrichtung zur Erzeugung von Lichtimpulsen ansteuert und die an der Kollektorfläche ankommenden Ladungsträger erfasst und daraus die Sauerstoffkonzentration ermittelt.

Die Erfindung beruht darauf, dass mittels kurzer Lichtimpulse, vorzugsweise aus LEDs oder Laserdioden, die Elektronen-Emitterfläche der Ionisationskammer bestrahlt wird, wodurch mittels der Photonen Elektronen von unterhalb der Fermi-Energie des Beschichtungsmaterials auf eine Energie geringfügig oberhalb der Energie im Vakuum angeregt werden, so dass diese die Schicht verlassen und sich frei im benachbarten Raum bewegen können. Dieser Raum im Inneren der Ionisationskammer bildet eine Driftröhre, die eine Anzahl von Potentialringen aufweist, die auf zunehmend höhere Potentiale gesetzt sind, wodurch die Elektronen in Richtung der der Elektronen-Emitterfläche gegenüber liegenden Kollektorfläche beschleunigt werden, wo die Elektronen aufgenommen und als elektrischer Strom messbar sind. Ins Innere der Ionisationskammer wird das Inertgas aus dem Treibstofftanks geführt, das im Wesentlichen aus molekularem Stickstoff (N₂) und enem gasförmigen Bestandteil des im Treibstofftank gelagerten Treibstoffs, meist Kerosin, besteht. Bei Kollisionen der Elektronen mit N₂-Molekülen werden diese von den Molekülen aufgrund deren negativer Elektronenaffinität nicht eingefangen, sondern geben lediglich ihre kinetische Energie an die N₂-Moleküle ab. Sofern das Gas jedoch Sauerstoff enthält, bleibt ein Teil der sich von der Emitterfläche zur Kollektorfläche bewegenden Elektronen an den Sauerstoffmolekülen gefangen und bilden O₂⁻-Ionen, die sich aufgrund der wesentlich höheren Masse viel langsamer zur Kollektorfläche bewegen. Die Bewegungsgeschwindigkeit eines O₂⁻Ions liegt etwa um den Faktor 100 niedriger als der eines Elektrons. Aus dem zeitlichen Verlauf des einem Lichtimpuls folgenden elektrischen Ladungsträgerstroms an der Kollektorfläche oder aus der Größe des gemessenen Stromes oder einer Kombination von beiden lässt sich die Sauerstoffkonzentration bestimmen.

Die Erfindung ermöglicht eine genaue Erfassung der Sauerstoffkonzentration in einer Inertgasatmosphäre, wobei der bauliche Aufwand durch die Verwendung einfacher Lichterzeugungsvorrichtungen wie LEDs oder Halbleiterlaser gering gehalten werden kann. Ferner ist eine Erhitzung einzelner Bestandteile der Vorrichtung nicht erforderlich.

Gemäß einer vorteilhaften Weiterbildung der Erfindung erzeugt die Lichtimpuls-Erzeugungsvorrichtung Lichtimpulse von 3 bis 15 nsec. Damit lassen sich mit geringen elektrischen Leistungen Spitzenleistungen im Bereich von 5 - 50 kW erzeugen, wodurch Elektronendichten im Bereich von ca. 10³ A/cm² erzielbar sind.

Gemäß einer vorteilhaften Weiterbildung der Erfindung erzeugt die Lichtimpuls-Erzeugungsvorrichtung Lichtimpulse einer Wellenlänge von 200 - 900 nm. Mittels kostengünstiger Halbleiterlichtquellen wie Laser oder LEDs im nahen Infrarotbereich von ca. 900 nm oder dem sichtbaren Bereich von ca. 700 nm bis 400 nm lassen sich ausreichend hohe Elektronendichten erreichen. Alternativ können auch UV-LEDs mit bis zu 250 nm Wellenlänge verwendet werden, wodurch Photonenenergien im Bereich von ca. 5 eV erzeugt werden können.

Gemäß einer vorteilhaften Weiterbildung der Erfindung ist die Lichtimpuls-Erzeugungsvorrichtung eine Laserdiodenanordnung oder eine LED-Anordnung. Lichtimpuls-Erzeugungsvorrichtungen sind kostengünstig und können aus einer oder auch mehreren Dioden bestehen.

Gemäß einer vorteilhaften Weiterbildung der Erfindung weist die Lichtimputs-Erzeugungsvorrichtung eine Leistung von 0,1 - 1 mW auf. Insbesondere bei kurzen Pulsen im Bereich von 15 nsec und weniger lassen sich ausreichend hohe Leistungen erzielen.

Gemäß einer vorteilhaften Weiterbildung der Erfindung besteht die Elektronen-Emitterfläche aus transparentem Saphirglas, das innenseitig mit einer Schicht aus einem Metall oder Halbleiter beschichtet ist. Das bewirkt vorteilhafterweise, dass verschiedene Emitter Materialien mit unterschiedlicher Austrittsarbeit verwenden können und dass die Bestrahlung zur Eletctronenauslösung sowohl in Reflexion als auch in Transmission erfolgen kann.

Gemäß einer vorteilhaften Weiterbildung der Erfindung ist die Metallschicht der Elektronen-Emitterfläche ausgewählt aus einem oder mehreren der folgenden Metalle: Au, Pt, Pd. Alle diese Metalle eignen sich aufgrund ihrer Elektronegativität von ca. 5 - 6 eV zur Emission von Photoelektronen. Am besten ist dabei Au aufgrund der geringen Oxidationsneigung.

Gemäß einer alternativen vorteilhaften Weiterbildung der Erfindung weist die Elektronen-Emitterfläche eine Schicht eines n-Halbleiters auf. Vorzugsweise eine Schicht aus BaO, weil Bariumoxid aufgrund ihrer Elektronegativität von ca. 1,4 eV auch mit Wellenlängen von bis zu 900 nm funktioniert, so dass kleine und hoch effiziente GaAs-Halbleiterlaser verwendet werden können.

Gemäß einer vorteilhaften Weiterbildung der Erfindung weist die Schicht der Elektronen-Emitterfläche Nanospitzen auf. Eine derartig konfigurierte Elektronen-Emitterfläche kann sowohl aus Metall als auch einem Halbleitermaterial bestehen. Nanospitzen eignen sich dazu, durch den quantenmechanischen Tunneleffekt die Austrittsarbeit für das Herauslösen von Elektronen auf nahezu Null zu reduzieren. Gemäß einer vorteilhaften Weiterbildung der Erfindung weisen dabei die Nanospitzen ein Verhältnis Abstand zu Spitzenradius von 100:1 bis 1000:1 auf. Vorzugweise haben die Nanospitzen einen Spitzenradius im Bereich von ca. 1 µm.

Gemäß einer vorteilhaften Weiterbildung der Erfindung weist die Ionisationskammer eine Länge zwischen Elektronen-Emitterfläche und Kollektorfläche von 3 bis 15 cm, vorzugsweise 5 bis 10 cm, auf. Mit modernen Lasern hoher Intensität lassen sich Elektronenstromdichten im Bereich von ca. 10³ A/cm² aus der Elektronen-Emitterfläche extrahieren, was andererseits nur bei Elektrodenspalte im Bereich von 100 µm funktioniert, Größere Längen der Ionisationskammer zur Auflösung von Elektronen/Ionen Driftspektren weisen deutlich geringere Elektronenstromdichten auf, die jedoch zu den Lichtleistungen von verfügbaren LED-Strahlungsquellen passen.

Gemäß einer vorteilhaften Weiterbildung der Erfindung weist die Vorrichtung eine Driftspannung von 2000 - 4000 V, vorzugsweise von 2500 - 3500V, auf.

Gemäß einer vorteilhaften Weiterbildung der Erfindung weist die Steuereinheit einen Tiefpassfilter für das Signal der Ladungsträger-Detektionsvorrichtung auf. Damit lässt sich vorteilhafterweise die Messgenauigkeit durch Eliminierung der Störsignale verbessern, wobei dabei die Zeitauflösung verschlechtert wird, was aber auf die Messergebnisse keine signifikanten Auswirkungen hat.

Vargeschlagen wird ferner ein Luftfahrzeug mit mindestens einem Treibstofftank, wobei der mindestens eine Treibstofftank eine Vorrichtung zur Überwachung der Sauerstoffkonzentration nach einer oder mehreren der vorbeschriebenen Ausführungsformen aufweist. Da moderne Großraumflugzeuge eine Vielzahl von Treibstofftanks aufweisen, können einzelne oder alle der Treibstofftanks entsprechend ausgerüstet werden. Vorzugsweise die im Rumpf befindlichen besonders gefährdeten Treibstofftanks sind entsprechend ausgerüstet.

Gemäß einer vorteilhaften Weiterbildung dieser Ausführung weist das Luftfahrzeug mehrere Treibstofftanks sowie mindestens eine Vorrichtung nach einer oder mehreren der vorbeschriebenen Ausführungsformen auf, die mit einem Gasleitungssystem gekoppelt ist, um gleichzeitig oder alternierend die Sauerstoffkonzentrationen in den einzelnen Treibstofftanks zu erfassen.

Weitere Vorteile, Merkmale und Einzelheiten ergeben sich aus der nachfolgenden Beschreibung, in der - unter Bezug auf die Zeichnung - ein Ausführungsbeispiel im Einzelnen beschrieben ist. Beschriebene und/oder bildlich dargestellte Merkmale bilden für sich oder in beliebiger, sinnvoller Kombination den Gegenstand der Erfindung. Gleiche, ähnliche und/oder funktionsgleiche Teile sind mit gleichen Bezugszeichen versehen.

Es zeigen:
- Figur 1:: eine schematische Darstellung einer Vorrichtung zur Überwachung der Sauerstoffkonzentration;
- Figur 2:: ein Diagramm, das einen zeitlichen Verlauf eines Messvorgangs mit unterschiedlichen Gaszusammensetzungen darstellt.

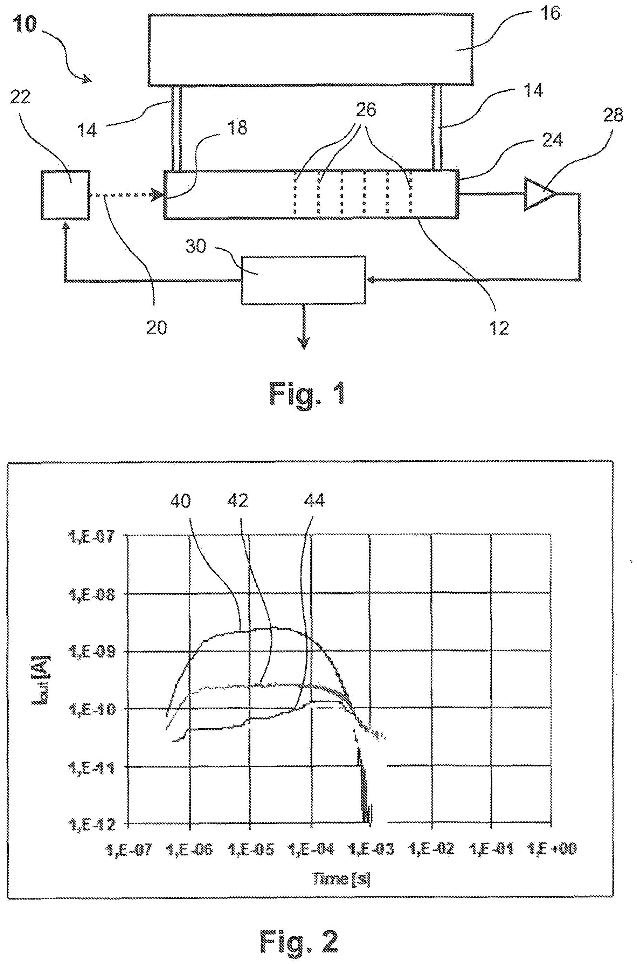

In **Figur 1** ist eine Sauerstoffmessvorrichtung 10 schematisch dargestellt. Diese umfasst eine Ionisationskammer 12, die über Verbindungsleitungen 14 und nicht dargestellte Fördereinrichtungen mit einem Treibstofftank 16 kommuniziert, so dass das im Treibstofftank 16 befindliche Gas mit der Ionisationskammer 12 zirkuliert. Die eine Stirnseite der aus Saphirglas gebildeten Ionisationskammer 12 ist als Elektronen-Emitterfläche 18 ausgebildet, d.h. innenseitig ist eine Beschichtung, vorzugsweise aus Au, aufgebracht, die von der Strahlung 20 einer außerhalb angeordneten Strahlungsquelle 22 beaufschlagt wird. Die Strahlungsquelle 22 ist vorzugsweise eine Laserdioden oder LED-Anordnung aus einer oder mehreren Lichtquellen.

Die der Emitterfläche 18 gegenüber liegende Stirnseite der Ionisationskammer 12 ist als Kollektor 24 ausgebildet. Die Ionisationskammer 12 umfasst ferner eine Felderzeugungseinrichtung zur Erzeugung eines elektrischen Feldes, vorzugsweise in Form mehrerer hinter einander angeordneten Potentialringen 26, die über nicht dargestellte Anschlüsse von der Emitterfläche 18 zur Kollektorfläche 24 hin mit zunehmenden Spannungen beaufschlagt sind. Die Spannung des hintersten Potentialrings liegt im Bereich von EUR 3000 V. Die Kollektorfläche 24 ist über einen Verstärker 28 mit einer Steuereinheit 30 gekoppelt. Mittels der Strahlungsquelle 22 werden kurze (ca. 5 nsec) Impulse von UV Licht erzeugt, die das UV-transparente Saphirglas der mit einer dünnen Goldschicht beschichteten Emitterfläche 18 durchdringen, und Elektronen von unterhalb der Fermi-Energie von Gold auf Energien oberhalb der Vakuumstufe angeregt werden, wo diese dann die Gold-Emitterschicht verlassen und sich frei im Raum bewegen können. Diese Elektronen aus der Emitterfläche 18 bewegen sich aufgrund der an den Potentialringen 26 anliegenden Spannung durch die Ionisationskammer 12 in Richtung der Kollektorfläche 24. An der Kollektorfläche 24 werden die Elektronen aufgenommen und als elektrischer Strom ertasst. Daraus lässt sich die Driftzeit der Elektronen durch die Ionisationskammer 12 bestimmen. Wenn sich in der Ionisationskammer 12 Umgebungsluft befindet, so wird ein Teil der emittierten Elektronen auf den Sauerstoffmolekülen eingefangen, wodurch sich relativ zu den Elektronen sehr langsam bewegende O₂⁻ Ionen erzeugt werden. Aus dem zeitlichen Verlauf des einem Lichtimpuls folgenden Stromes lässt sich der Sauerstoffgehalt bestimmen.

**Figur 2** zeigt Diagramm, das einen zeitlichen Verlauf eines Messvorgangs unter Verwendung eines Tiefpassfilters darstellt. Die Abszisse zeigt logarithmisch die Zeit seit Erzeugung eines Lichtimpulses in Sekunden, die Ordinate logarithmisch den gemessenen Strom in Ampere. Die oberste Kurve 40 ergibt sich bei einer reinen N₂-Atmosphäre, also ohne Sauerstoff, die mittlere Kurve 42 ergibt sich bei einem Gemisch aus N₂ mit normaler Luft im Volumenverhältnis von 50 vol-% N₂ und 50 vol-% normale Luft Die unterste Kurve 44 ergibt sich bei normaler Luft, die etwa zu 21 vol-% molekularen Sauerstoff enthält. Anhand der deutlich unterschiedlichen Maximalströme lässt sich der Sauerstoffgehalt im Inneren der Ionisationskammer 12 bestimmen. Wenngleich durch den verwendeten Tiefpassfilter eine Auflösung des Strommaximums bzw. dessen zeitliche Verschiebung durch die Präsenz von Sauerstoff nicht mehr messbar ist, so ergeben sich relativ breite Stromplateaus unterschiedliche Niveaus im Bereich von ca. 10 µsec. abhängig vom Sauerstoffgehalt der Messatmosphäre. Es sei angemerkt, dass sich die Messwerte gemäß Figur 2 nicht wesentlich ändern, wenn die Messatmosphäre zusätzlich die im Kerosin enthaltenen Kohlenwasserstoffe mit 6 bis 16 C-Atomen enthält, weil diese Elektronenaffinitäten aufweisen, die deutlich kleiner sind als die von Sauerstoff. Sofern die Messatmosphäre das erheblich elektronegative Molekül C₆F₆ enthält, führt dies zu einer Streckung des zeitlichen Strommaximumverlaufes, was erfassbar ist. Selbst wenn eine solche Analyse nicht durchgeführt wird, so führt das Vorhandensein solcher Moleküle in signifikanten Mengen nur zu einer Fehlmessung in Richtung eines erhöhten Sauerstoffgehaltes, also keinesfalls zu einer Erhöhung der Gefährdung, was nur bei Anzeige eines zu niedrigen Sauerstoffgehaltes der Fall wäre.

### Bezugszeichenliste

- 10: Sauerstoffmessvorrichtung
- 12: Ionisationskammer
- 14: Verbindungsleitungen
- 16: Treibstofftank
- 18: Elektronen-Emitterfläche
- 20: Strahlung
- 22: Strahlungsquelle
- 24: Kollektor
- 26: Potentialringe
- 28: Verstärker
- 30: Steuereinheit
- 40: N2-Atmosphäre
- 42: N2+ O2-Atmosphäre
- 44: Luft-Atmosphäre

## Patentansprüche

1. Luftfahrzeug mit mindestens einem ein Inertgas enthaltenden Treibstofftank (16), der eine Vorrichtung (10) zur Überwachung der Sauerstoffkonzentration in dem Treibstofftank (16) umfasst, **gekennzeichnet durch** folgende Merkmale:
- eine Ionisationskammer (12), die mit dem Treibstofftank (16) kommuniziert und von einem Inertgasstrom aus dem Treibstofftank (16) durchströmbar ist;
- wobei ein Ende der Ionisationskammer (12) eine Elektronen-Emitterfläche (18) aufweist;
- wobei die Ionisationskammer (12) eine der Elektronen-Emitterfläche (18) gegenüberliegende Kollektorfläche (24) aufweist, die mit einer Ladungsträger-Detektionsvorrichtung (28, 30) gekoppelt ist;
- eine Lichtimpuls-Erzeugungsvorrichtung (22) zur Bestrahlung der Elektronen-Emitterfläche (18) zur Erzeugung von Elektronen in der Ionisationskammer (12);
- wobei die Ionisationskammer (12) von einer Felderzeugungseinrichtung (26) umgeben ist zur Erzeugung eines elektrischen Feldes, um negative Ladungsträger in Richtung zur Kollektorfläche (24) zu bewegen;
- eine Steuereinheit (30) zur Ansteuerung der Lichtimpuls-Erzeugungsvorrichtung (22) zwecks Erzeugung von Lichtimpulsen und zur Erfassung von an der Kollektorfläche (24) ankommenden Ladungsträgern und daraus Ermittlung der Sauerstoffkonzentration.

2. Luftfahrzeug nach Anspruch 1, **dadurch gekennzeichnet, dass** die Lichtimpuls-Erzeugungsvorrichtung (22) Lichtimpulse von 3 bis 15 nsec erzeugt.

3. Luftfahrzeug nach Anspruch 1, **dadurch gekennzeichnet, dass** die Lichtimpuls-Erzeugungsvorrichtung (22) Lichtimpulse einer Wellenlänge von 200 - 900 nm erzeugt.

4. Luftfahrzeug nach Anspruch 1, **dadurch gekennzeichnet, dass** die Lichtimpuls-Erzeugungsvorrichtung (22) als Laserdiodenanordnung oder LED-Anordnung ausgebildet ist.

5. Luftfahrzeug nach Anspruch 1 oder 4, **dadurch gekennzeichnet, dass** die Lichtimpuls-Erzeugungsvorrichtung (22) eine Leistung von 0,1 - 1 mW aufweist.

6. Luftfahrzeug nach Anspruch 1, **dadurch gekennzeichnet, dass** die Elektronen-Emitterfläche (18) aus transparentem Saphirglas besteht, das innenseitig mit einer Schicht aus einem Metall oder Halbleiter beschichtet ist.

7. Luftfahrzeug nach Anspruch 6, **dadurch gekennzeichnet, dass** die Metallschicht der Elektronen-Emitterfläche (18) ausgewählt ist einem oder mehreren der folgenden Metalle: Au, Pt, Pd.

8. Luftfahrzeug nach Anspruch 6, **dadurch gekennzeichnet, dass** die Halbleiterschicht der Elektronen-Emitterfläche (18) ein n-Halbleiter, insbesondere BaO, ist.

9. Luftfahrzeug nach Anspruch 6, **dadurch gekennzeichnet, dass** die Schicht der Elektronen-Emitterfläche (18) Nanospitzen aufweist.

10. Luftfahrzeug nach Anspruch 9, **dadurch gekennzeichnet, dass** die Nanospitzen ein Verhältnis Abstand zu Spitzenradius von 100:1 bis 1000:1 aufweisen.

11. Luftfahrzeug nach Anspruch 1, **dadurch gekennzeichnet, dass** die Ionisationskammer (12) eine Länge zwischen Elektronen-Emitterfläche und Kollektorfläche von 3 bis 15 cm, vorzugsweise 5 bis 10 cm, aufweist.

12. Luftfahrzeug nach Anspruch 1, **dadurch gekennzeichnet, dass** diese eine Driftspannung von 2000 - 4000 V aufweist.

13. Luftfahrzeug nach Anspruch 1, **dadurch gekennzeichnet, dass** die Steuereinheit (30) einen Tiefpassfilter für das Signal der Ladungsträger-Detektionsvorrichtung aufweist.

14. Luftfahrzeug nach Anspruch 1 mit mehreren Treibstofftanks, **dadurch gekennzeichnet, dass** mindestens eine Vorrichtung (10) nach einem oder mehreren der vorherigen Ansprüche vorgesehen ist, die mit einem Gasleitungssystem gekoppelt ist, um gleichzeitig oder alternierend die Sauerstoffkonzentrationen in den einzelnen Treibstofftanks zu erfassen.

## Claims

1. Aircraft having at least one fuel tank (16) that contains an inert gas, said fuel tank comprising a device (10) for monitoring the concentration of oxygen in the fuel tank (16), **characterized by** means of the following features:
- an ionisation chamber (12) that communicates with the fuel tank (16) and it is possible for an inert gas flow to flow through said ionisation chamber from the fuel tank (16),
- wherein one end of the ionisation chamber (12) comprises an electron emitter surface (18),
- wherein the ionisation chamber (12) comprises a collector surface (24) that lies opposite the electron emitter surface (18), said collector surface being coupled to a charge carrier detecting device (28, 30),
- a light pulse generating device (22) for irradiating the electron emitter surface (18) so as to generate electrons in the ionisation chamber (12),
- wherein the ionisation chamber (12) is surrounded by a field-generating device (26) for generating an electrical field in order to move negative charger carriers in the direction of the collector surface (24),
- a control unit (30) for controlling the light pulse generating device (22) for the purpose of generating light pulses and for ascertaining charge carriers that arrive on the the collector surface (24) and to determine therefrom the concentration of oxygen.

2. Aircraft according to Claim 1, **characterized in that** the light pulse generating device (22) generates light pulses of 3 to 15 nanoseconds.

3. Aircraft according to Claim 1, **characterized in that** the light pulse generating device (22) generates light pulses of a wave length of 200 - 900 nm.

4. Aircraft according to Claim 1, **characterized in that** the light pulse generating device (22) is embodied as a laser diode arrangement or LED arrangement.

5. Aircraft according to Claim 1 or 4, **characterized in that** the light pulse generating device (22) comprises a power of 0.1 - 1 mW.

6. Aircraft according to Claim 1, **characterized in that** the electron emitter surface (18) is embodied from transparent sapphire glass that is coated on the inner face with a coating of metal or semi conductor.

7. Aircraft according to Claim 6, **characterized in that** the electron emitter surface (18) is selected from one or multiple of the following metals: Au, Pt, Pd.

8. Aircraft according to Claim 6, **characterized in that** the semiconductor coating of the electron emitter surface (18) is an n-semiconductor, in particular BaO.

9. Aircraft according to Claim 6, **characterized in that** the coating of the electron emitter surface (18) comprises nanotips.

10. Aircraft according to Claim 9, **characterized in that** the nanotips comprise a ratio distance of 100:1 to 1000:1 with respect to the tip radius.

11. Aircraft according to Claim 1, **characterized in that** the ionisation chamber (12) comprises a length between the electron emitter surface and the collector surface of 3 to 15 cm, preferably 5 to 10 cm.

12. Aircraft according to Claim 1, **characterized in that** said aircraft comprises a drift voltage of 2000 - 4000 V.

13. Aircraft according to Claim 1, **characterized in that** the control unit (30) comprises a low-pass filter for the signal of the charge carrier detecting device.

14. Aircraft according to Claim 1 having multiple fuel tanks, **characterized in that** at least one device (10) according to any one or multiple of the preceding claims is provided, said device being coupled to a gas line system in order to simultaneously or on an alternating basis ascertain the concentration of oxygen in the individual fuel tanks.

## Revendications

1. Aéronef comprenant au moins un réservoir à carburant (16) contenant un gaz inerte, lequel réservoir à carburant comprend un dispositif (10) de surveillance de la concentration en oxygène dans le réservoir à carburant (16), **caractérisé par** les caractéristiques suivantes :
- une chambre d'ionisation (12) qui communique avec le réservoir à carburant (16) et peut être traversée par un courant de gaz inerte en provenance du réservoir à carburant (16) ;
- dans lequel, une extrémité de la chambre d'ionisation (12) présente une surface d'émetteurs d'électrons (18) ;
- dans lequel la chambre d'ionisation (12) présente une surface de collecteurs (24) opposée à la surface d'émetteurs d'électrons (18), qui est couplée à un dispositif de détection (28, 30) de porteurs de charge ;
- un dispositif générateur d'impulsions lumineuses (22) destiné à irradier la surface d'émetteurs d'électrons (18) pour générer des électrons dans la chambre d'ionisation (12) ;
- dans lequel la chambre d'ionisation (12) est entourée par un dispositif générateur de champ (26) destiné à générer un champ électrique afin de déplacer des porteurs de charge négatifs en direction de la surface de collecteurs (24) ;
- une unité de commande (30) destinée à commander le dispositif générateur d'impulsions lumineuses (22) dans le but de générer des impulsions lumineuses et de détecter les porteurs de charge arrivant sur la surface de collecteurs (24) et, à partir de ceci, de déterminer la concentration d'oxygène.

2. Aéronef selon la revendication 1, **caractérisé en ce que** le dispositif générateur d'impulsions lumineuses (22) génère des impulsions lumineuses de 3 à 15 nsec.

3. Aéronef selon la revendication 1, **caractérisé en ce que** le dispositif générateur d'impulsions lumineuses (22) génère des impulsions lumineuses d'une longueur d'onde de 200 à 900 nm.

4. Aéronef selon la revendication 1, **caractérisé en ce que** le dispositif générateur d'impulsions lumineuses (22) est réalisé en tant que agencement à diodes laser ou agencement à LED.

5. Aéronef selon la revendication 1 ou 4, **caractérisé en ce que** le dispositif générateur d'impulsions lumineuses (22) présente une puissance de 0,1 à 1 mW.

6. Aéronef selon la revendication 1, **caractérisé en ce que** la surface d'émetteurs d'électrons (18) est constituée de verre saphir transparent qui est recouvert côté intérieur par une couche constituée d'un métal ou d'un semi-conducteur.

7. Aéronef selon la revendication 6, **caractérisé en ce que** la couche métallique de la surface d'émetteurs d'électrons (18) est choisie à partir d'un ou de plusieurs des métaux suivants : Au, Pt, Pd.

8. Aéronef selon la revendication 6, **caractérisé en ce que** la couche de semi-conducteur de la surface d'émetteurs d'électrons (18) est un semi-conducteur n, notamment BaO.

9. Aéronef selon la revendication 6, **caractérisé en ce que** la couche de la surface d'émetteurs d'électrons (18) présente des nanopointes.

10. Aéronef selon la revendication 9, **caractérisé en ce que** les nanopointes présentent un rapport écart/rayon de pointe de 100 :1 à 1000 :1.

11. Aéronef selon la revendication 1, **caractérisé en ce que** la chambre d'ionisation (12) présente une longueur entre la surface d'émetteurs d'électrons et la surface de collecteurs de 3 à 15 cm, de préférence de 5 à 10 cm.

12. Aéronef selon la revendication 1, **caractérisé en ce que** celle-ci présente une tension de dérive de 2000 à 4000 V.

13. Aéronef selon la revendication 1, **caractérisé en ce que** l'unité de commande (30) présente un filtre de passe-bas pour le signal du dispositif de détection de porteurs de charge.

14. Aéronef selon la revendication 1 comprenant plusieurs réservoirs à carburants, **caractérisé en ce qu'**au moins un dispositif (10) selon une ou plusieurs des revendications précédentes est ménagé, lequel est couplé à un système de conduites de gaz afin de détecter simultanément ou en alternance les concentrations d'oxygène dans chacun des réservoirs à carburant.
